# EUROPEAN PATENT SPECIFICATION

(11) **EP 2 903 590 B1**
(45) Date of publication and mention of the grant of the patent: **16.06.2021**
(21) Application number: 13786146.4
(22) Date of filing: 01.10.2013
(51) Int. Cl.: A61K 36/889, A61P 17/02, A61P 17/00

(54) **PLANT LIPID COMPOSITION FOR MODULATING FUNCTIONS OF KERATINOUS MATERIALS, METHOD FOR MODULATING SAID FUNCTIONS AND USE OF SAID PLANT LIPIDS**
ZUSAMMENSETZUNG AUS PFLANZLICHEN LIPIDEN ZUR MODULATION DER FUNKTIONEN EINES KERATINMATERIALS, VERFAHREN ZUR MODULATION DIESER FUNKTIONEN UND VERWENDUNG BESAGTER PFLANZLICHER LIPIDE
COMPOSITION DE LIPIDES VÉGÉTAUX POUVANT MODULER DES FONCTIONS DE MATIÈRES KÉRATINIQUES, PROCÉDÉ DE MODULATION DESDITES FONCTIONS, ET UTILISATION DE CES LIPIDES VÉGÉTAUX

(30) Priority: 01.10.2012 US 201261708266 P
(43) Date of publication of application: 12.08.2015
(73) Proprietor: Natura Cosméticos S.A., 06882-700 Sao Paulo (BR)
(72) Inventor: FERRARI, Cintia Rosa, 06390-500 São Paulo - SP (BR); ZIMBARDI, Daniela, 13450-182 Santa Barbara d'Oeste - SP (BR); PLACONÁ DINIZ, Gabriela, 04810-110 São Paulo - SP (BR); DE ASSIS SANTOS, Ícaro, 13209-355 Jundiaí São Paulo (BR); LORENCINI, Marcio, 80210-390, Curitiba - PR (BR); DA LUZ MOREIRA, Patrícia, 05051-030 São Paulo - SP (BR)
(74) Representative: Simmons & Simmons
(86) International application number: PCT/BR2013/000388
(87) International publication number: WO 2014/053038

(56) References cited:
- EP-A1- 2 335 685
- WO-A1-2008/156627
- WO-A2-2007/062206
- DE-A1-102008 012 058
- DE-U1-202008 014 069
- US-A1- 2007 166 253
- US-A1- 2007 166 275
- US-A1- 2009 285 876
- US-A1- 2011 159 121
- DATABASE GNPD [Online] MINTEL; February 2009 (2009-02), Nature & Decouvertes: "Face Care Elixir", XP002722672, Database accession no. 1063394
- DATABASE GNPD [Online] MINTEL; October 2007 (2007-10), LH Skincare: "Body Oil", XP002722673, Database accession no. 781289

## Description

### Field of the Invention

The present invention is directed to compositions for use in tissue healing and/or repair comprising at least one plant lipid, having as main application the treatment of keratinous materials, particularly skin and scalp by modulating the functions of said keratinous materials through the modulation of the expression of genes involved in these functions.

### Background of the Invention

With the action of time and aging, the skin suffers from a reduction of both the structural proteins essential for its support and of the substances in which these fibers are layered, resulting in loss of firmness and filling. Several biological processes contribute to the aging of keratinous material; each of these processes is regulated by the expression of specific genes. Lipids are a group consisting of several organic compounds found in animals, plants and microorganisms that possess important energetic and metabolic functions in the organism. Due to the diversity of these compounds, lipids have broad applications in nutrition and in developing cosmetics and pharmaceuticals, and they can also be used in consumer goods industries (paints, detergents, etc.).

Triglycerides, also known as esters of trihydroxy alcohols with three fatty acid molecules attached, are the major lipids found in vegetable oils and butters, which are widely used raw materials. Vegetable oils and butters are water-insoluble substances whose compositions and proportions of triglycerides and fatty acids vary with the species of origin of the material. The main difference between vegetable oils and butters is given by their state at room temperature: oils are liquid and butters are solid. These characteristics are strongly related to the composition of fatty acids in their triglyceride molecules (Alvarez and Rodriguez, 2000).

The vegetable oils and butters are commonly used in cosmetic and pharmaceutical formulations as emollients, which are agents that moisturize the skin by promoting a reduction of transepidermal water loss (TEWL) (Leyden and Rawlings, 2002). However, there are very few scientific studies that show a biological effect promoted by vegetable oils and butters when inserted into topical compositions.

EP 2 335 685 A1 concerns a skin-whitening agent, anti-aging agent and skin cosmetic. US 2007/166253 A1 concerns cosmetic and dermatological formulations with natural pigments. US 2009/285876 A1 concerns natural butters reconstituted by transesterification with glycerin and its use in cosmetics applications. WO 2007/062206 A2 concerns chocolate formulations with functional properties. DE 20 2008 014069 concerns cosmetic composition for topical use. US 2007/166275 A1 concerns compositions comprising kakadu plum extract or acai berry extract. Mintel; February 2009 (2009-02), Nature & Decourvertes: "Face Care Elixir" concerns a skincare composition containing acai oil amongst other ingredients. Mintel October 2007 (2007-10), LH Skincare: "Body Oil" concerns a body care composition containing acai oil amongst other ingredients. US 2011/159121 A1 concerns methods and compositions for identifying, producing and using plant-derived products for modulating cell function and aging. WO 2008/156627 A1 concerns phytochemical-rich oils and methods related thereto. DE 10 2008 012058 A1 concerns hair treatment with acai extract.

The Applicant, through extensive scientific research, has found that plant lipids act directly on genes related to the functionality and maintenance of keratinous materials.

Accordingly, the regulation of the expression of these genes via plant lipids represents a particularly novel technological approach for the treatment of keratinous materials.

### Brief Description of the Invention

The present invention provides a composition for use in tissue healing and/or repair according to claim 1.

### Description of Drawings

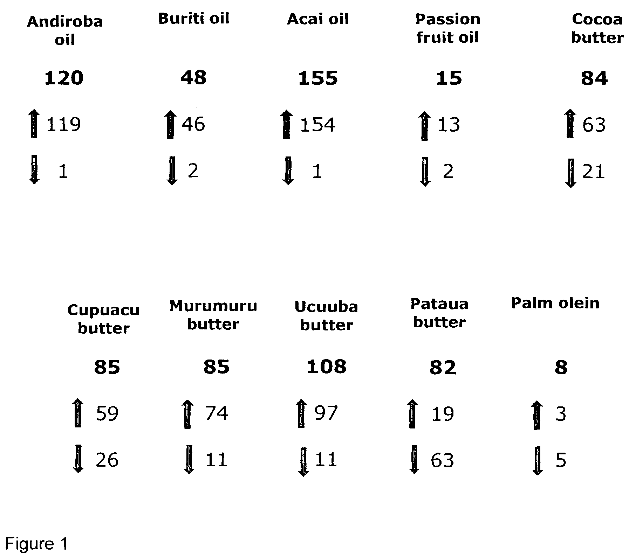

Figure 1 is a representative schematic showing the number of genes modulated by different plant lipids as disclosed in the present invention. The upwards arrows indicate the number of genes that had their expression increased relative to the control (control = 1), while the downward arrows compared to the respective control (control = 1).

### Detailed Description of the Invention

In the context of the present invention, which is directed to the therapeutic use of the material as defined in claim 1, "keratinous materials" means skin and scalp.

The amount of plant lipids in the compositions of the present invention naturally depends on the desired cosmetic or pharmaceutical effect and thus may vary widely.

In a particular embodiment, the at least one plant lipid is present in the cosmetic composition of the present invention in an amount ranging from about 0.01% to about 50% by weight, preferably from about 0.5% to about 20 wt%, more preferably about 1 % to about 5% by weight, the amounts based on the total weight of the composition.

### Compositions

The compositions of the present invention may be ingested, injected or applied directly to the keratinous materials. Depending on the route of administration, the compositions of the present invention may be provided in the galenic and cosmetic forms normally used.

For topical application to the keratinous materials, the compositions of the present invention may be in the form of aqueous, alcoholic or oily solutions, dispersions, emulsions, suspensions, aerosol compositions comprising pressurized propellant agent, microcapsules, microparticles or ionic or non-ionic type vesicular dispersions.

More specifically, by way of example and without limitation, one can mention gels, lotions in general, such as aftershave and fasteners, foams, creams, such as lotions for cleansing, protection, treatment or care of the face, hair (e.g. anti-aging cream and combing cream) or other body regions, makeup removers, such as foundations, lipsticks, face masks, nail polishes, eyeliners, shadows, powders, among other forms known by skilled artisans, sunscreens, milks, mists, oils, butters, bar soaps, liquid rinse soaps or non-rinse soaps, shampoos, conditioners, styling products, restructurers, moisturizers, deodorants, sunscreens, depilatory creams, bath water, makeup, aerosol composition comprising pressurized propellant agent, dye compositions, compositions for permanent waving or straightening and compositions for scalp treatment.

For injectable application, the compositions of the present invention may be provided in the form of an aqueous or oily lotion or in the form of serum.

For oral application, said compositions may be provided in the form of capsules, tablets, granules, syrups, or any other pharmaceutical form known to those skilled in the art.

The quantities of the various constituents of the compositions of the present invention are those conventionally used in the fields considered and are not limitative of the present invention.

The compositions of the present invention are prepared in ways known to those skilled in the art.

### Optional Components

To provide the cosmetic composition and the pharmaceutical composition of the present invention with some desirable characteristic not yet achieved with the aforementioned components, optional components may be added which are compatible with the properties thereof. Some of the components that may be added to said compositions are listed below.

A chelating agent may be added to the compositions of the present invention, for example, amino acids, EDTA, dimercaprol and etidronic acid. Said chelating agents may be added at a concentration range between 0.10 and 0.50% of the composition.

The following examples of emulsifiers can be mentioned: alkoxylated carboxylic acids, phosphorus compounds, fatty acids, such as, without limitation, a mixture of glyceryl stearate/PEG-100 stearate, potassium cetyl phosphate, glycerol stearate and polysorbates, for example, polysorbate 60. Said emulsifiers may be present at a concentration of between 1 and 5%.

As examples of moisturizers, biological polymers and derivatives, and also alcohols, such as glycerin and propylene glycol may be mentioned. Said moisturizers may be present at a concentration of between 1 and 10%.

As sunscreens, physical or chemical filters may be added. Examples of sunscreens that may be used in the composition of the present invention are ethylhexyl salicylate, ethylhexyl methoxycinnamate, methyl 4-methoxycinnamate, ethyl 4-methoxycinnamate, octyl 4-methoxycinnamate, octocrylene, benzophenone-3, diethylamino hydroxybenzoyl hexyl benzoate, avobenzone, phenylbenzimidazole sulfonic acid, dietilhexilbutamido triazone, diethanolamide methoxycinnamate, homosalate, ethylhexyl triazone, polysilicon-15, benzoxazoylphenyl ethylhexylamine bis-triazine, 2,6-diethylhexyl naphthalate, butyl methoxydibenzoylmethane, mixture of titanium dioxide, silica and dimethicone (Parsol TX), bisoctrizol (Tinosorb M), bemotrizinol (Tinosorb S), disodium phenyl dibenzimidazol tetrasulphonate (Neo Heliopan AP) or a mixture of one or more thereof. Said filters may be present in the compositions of the present invention at a concentration of between 5 and 40%.

As examples of emollients, esters, fatty acids, alcohols, plant polymers, ethers, oils and fats, such as alkyl benzoate, cetyl lactate, cetiol sensoft, dicaprilyl carbonate, isononyl isononanoate, macadamiate acetate, caprylic/capric triglyceride, pentanediol, palm olein, hexylene glycol may be mentioned. Said emollients may be present at a concentration comprised between 1 and 10%.

As consistency agents, fatty alcohols, for example, cetyl alcohol and cetostearyl alcohol may be mentioned. Said consistency agents may be present at a concentration comprised between 0.2 and 5%.

As thickeners/viscosity agents, carbomers, polymers, gums and salts, for example, alkyl acrylate, Carbopol, xanthan gum and sodium chloride may be added. Said thickeners may be present at a concentration comprised between 0.2 and 5%.

As sensory modifiers, silicones, starches, synthetic polymers and minerals may be added. Examples of sensory modifiers are D5/D6 cyclomethicone, cyclomethicone and dimethicone crospolymer, tapioca starch, nylon, silica, among others. Said modifiers are present in the compositions of the present invention at a concentration of between 1 and 8%.

As an antioxidant, for example, phenols such as BHT, BHA, vitamin C, OPC from grape seeds, or even an antioxidant complex may be added.

In a particular embodiment, an antioxidant complex developed by the applicant, consisting of the combination of coffee extract, lycopene and vitamin E is added.

In another particular embodiment, the antioxidant complex developed by the applicant, consisting of the combination of green tea extract, dry cocoa extract and tocopheryl acetate is added.

Said antioxidant may be present at a concentration between 0.1 and 0.5% of the composition.

As oils and waxes that can be used in the composition of the present invention vaseline, shea butter, sunflower oil, perhydrosqualene, purcelin oil, silicone oil, cyclomethicone, beeswax, carnauba wax, paraffin, among others. As an example of solvents water, oils or lower alcohols, especially ethanol and isopropanol and propylene glycol.

As gelling agents, hydrophilic gelling agents may be added, such as carboxyvinyl polymers (carbomer), acrylic copolymers, such as acrylate/alkyl acrylate copolymers, polyacrylamides, polysaccharides, such as hydroxypropylcellulose, naturally-occurring gums and clays. As lipophilic gelling agents, bentones, metal salts of fatty acids may be mentioned, such as aluminum stearates and hydrophobic silica, ethylcellulose and polyethylene.

As preservative agent, alcohols and heterocyclic compounds may be added, such as phenoxyethanol, methylchloroisothiazolinone and methylisothiazolinone. Said preservative agent may be present in the compositions of the present invention at a concentration comprised between 0.01 and 2%.

As pH correction agents, alkanolamines and inorganic bases may be added, such as triethanolamine, sodium hydroxide, and others known to the person skilled in the art. Said correction agents are added in an amount sufficient to achieve the desired pH.

Conditioning agents may also be added, such as dimethicone, dimethiconol TEA or a combination of dimethiconol and TEA dodecylbenzenesulphonate. Conventional surfactants may also be added, such as cocoamido propyl betaine, decyl polyglucose and sodium lauryl ether sulfate.

Pearlizing agents may also be added, such as glycol distearate, ethylene glycol distearate or diethylene glycol distearate.

### Optional Additional Actives

If desired, additional actives may be added to the cosmetic and/or pharmaceutical compositions of the present invention. Among them, the following actives may be mentioned, for example:
- proteins and protein hydrolysates, amino acids, urea, allantoin, sugars and sugar derivatives, such as biosaccharide gum, particularly biosaccharide gum 3, vitamins such as retinol (vitamin A), tocopherol (vitamin E) and derivatives thereof, alcohols and plant extracts, such as, without limitation, Passiflora alata extract, green tea, grape OPC, cocoa extract, jambu extract, essential oil of pitanga, among others, essential fatty acids, ceramides, essential oils, salicylic acid and derivatives thereof;

- modulating agents of skin pigmentation and/or proliferation and/or differentiation, such as retinoic acid and its isomers, retinol and its esters, vitamin D and its derivatives, estrogens, such as estradiol, levonorgestrel, ethinyl estradiol, kojic acid and hydroquinone;
- antibacterial agents, such as clindamycin phosphate, erythromycin, or antibiotics of the tetracycline class;
- antiparasitic agents, in particular metronidazole, crotamiton or pyrethroids;
- antifungal agents, especially compounds of the imidazole class, such as econazole, ketoconazole or miconazole and salts thereof, polyene compounds, such as amphotericin B, compounds of the allylamine family, such as terbinafine, or alternatively octopirox;
- antiviral agents, such as acyclovir;
- steroidal anti-inflammatory agents, such as hydrocortisone, betamethasone valerate, clobetasol propionate or non-steroidal anti-inflammatory agents, such as ibuprofen and salts thereof, celecoxib and salts thereof, diclofenac and salts thereof, acetylsalicylic acid, acetaminophen or glycyrrhetinic acid;
- anesthetic agents, such as lidocaine hydrochloride and derivatives thereof;
- antipruritic agents, such as thenaldine, trimeprazine or cycloheptadine;
- keratolytic agents, such as alpha- and beta-hydroxycarboxylic acids or beta-keto carboxylic acids, their salts, amides or esters, glycolic acid, lactic acid, salicylic acid, citric acid, n-octanol-5-salicylic acid, among others.
- anti-free radical agents, such as alpha-tocopherol and esters thereof, superoxide dismutases, metallic chelating agents or ascorbic acid and esters thereof;
- antiseborrheic agents, such as progesterone;
- antidandruff agents, such as zinc pyrithione and octopirox;
- antiacne agents, such as retinoic acid and benzoyl peroxide;
- anti hair loss agents, such as minoxidil;
- antilipemic agents, such as atorvastatin, simvastatin, pravastatin;
- antidrepressive agents/muscle relaxant agents, such as, without limitation, venlafaxine, lorazepam, gabapentin;
- anti-acid secretion agents, such as omeprazole, lansoprazole, dexlansoprazole, esomeprazole, pantoprazole, rabeprazole;
- diuretic agents, such as hydrochlorothiazide, sildenafil citrate.

### Method

Also disclosed herein is a method of cosmetic or therapeutic treatment comprising the steps of:
a) Separating an effective amount of the cosmetic or therapeutic composition of the present invention;
b) Applying the effective amount in the region expected to receive the treatment.

Preferably, the method of cosmetic or therapeutic treatment comprises modulating the function of ROS metabolism, lipid metabolism, skin integrity, cell renewal, cell differentiation, strengthening the skin barrier, calcium metabolism, cell proliferation, tissue healing and/or repair, cell adhesion, carbohydrate metabolism, hydration, skin homeostasis/maintenance, skin defense and/or protection, remodelling and/or extracellular matrix synthesis, skin peeling, tissue maintenance, cell signaling, normal development of the epidermis, epidermal barrier function, skin development, the dermis-epidermis junction, anti-inflammatory or antioxidant action in keratinous materials of the skin and scalp, by administering a composition as defined in the present invention. The person skilled in the art will know how to choose the optimum composition for the desired end in the teachings disclosed in the present specification.

### Use

Also disclosed herein is a use of a plant lipid for the preparation of a cosmetic or pharmaceutical composition for treating keratinous materials through the modulation of the expression of specific genes related to the functioning and/or maintenance of keratinous materials.

### Genes modulated by the compositions of the present invention

Table 1 lists the genes the expression of which is modulated by the compositions of the present invention.

**Table 1**

| **SEQ ID NO:** | **Gene Symbol** | **Gene Name** |
|---|---|---|
| 1 | DSC2 | desmocollin 2 |
| 2 | GJA1 | gap junction protein, alpha 1, 43kDa |
| 3 | GJB2 | gap junction protein, beta 2 |
| 4 | IGF1R | insulin-like growth factor 1 receptor |
| 5 | ITGA1 | integrin, alpha 1 |
| 6 | ITGB1 | integrin, beta 1 (fibronectin receptor, beta polypeptide, antigen CD29 includes MDF2, MSK12 |
| 7 | ITGB2 | integrin, beta 2 (complement component 3 receptor 3 and 4 subunit) |
| 8 | ITGB6 | integrin, beta 6 |
| 9 | LAMB1 | laminin, beta 1 |
| 10 | LAMB3 | laminin, beta 3 |
| 11 | CAT | catalase |
| 12 | GPX1 | glutathione peroxidase 1 |
| 13 | MSRA | methionine sulfoxide reductase A |
| 14 | MSRB2 | methionine sulfoxide reductase B2 |
| 15 | NOX1 | NADPH oxidase 1 |
| 16 | NOX5 | NADPH oxidase, EF-hand calcium binding domain 5 |
| 17 | NR1H2,hCG22944 | nuclear receptor subfamily 1, group H, member 2 |
| 18 | PRDX6 | peroxiredoxin 6 |
| 19 | RARB | retinoic acid receptor, beta |
| 20 | S100A12 | S100 calcium binding protein A12 |
| 21 | DEFB1 | defensin, beta 1 |
| 22 | DEFB103A, | defensin, beta 103A |
| 23 | DEFB103B | defensin, beta 103B |
| 24 | DEFB4A | defensin, beta 4A |
| 25 | CAMP | cathelicidin antimicrobial peptide |
| 26 | CDH1 | cadherin 1, type 1, E-cadherin (epithelial) |
| 27 | CDSN | corneodesmosin |
| 28 | CGN | cingulin |
| 29 | CLDN1 | claudin 1 |
| 30 | CLDN4 | claudin 4 |
| 31 | CLDN5 | claudin 5 |
| 32 | CLDN7 | claudin 7 |
| 33 | COL17A1 | collagen, type XVII, alpha 1 |
| 34 | CTNNA1 | catenin (cadherin-associated protein), alpha 1, 102kDa |
| 35 | DSG4 | desmoglein 4 |
| 36 | DSP | desmoplakin |
| 37 | DST | dystonin |
| 38 | ELOVL3 | elongation of very long chain fatty acids (FEN1/Elo2, SUR4/Elo3, yeast)-like 3 |
| 39 | F11R | F11 receptor |
| 40 | GBA | glucosidase, beta, acid |
| 41 | ITGA6 | integrin, alpha 6 |
| 42 | ITGB4 | integrin, beta 4 |
| 43 | JUP | junction plakoglobin |
| 44 | KRT1 | keratin 1 |
| 45 | KRT10 | keratin 10 |
| 46 | KRT14 | keratin 14 |
| 47 | KRT15 | keratin 15 |
| 48 | KRT16 | keratin 16 |
| 49 | KRT17 | keratin 17 |
| 50 | KRT19 | keratin 19 |
| 51 | KRT4 | keratin 4 |
| 52 | KRT5 | keratin 5 |
| 53 | KRT6A | keratin 6A |
| 54 | KRT7 | keratin 7 |
| 55 | LAMA5 | laminin, alpha 5 |
| 56 | OCLN | Occludin |
| 57 | PKP1 | plakophilin 1 (ectodermal dysplasia/skin fragility syndrome) |
| 58 | PPHLN1 | periphilin 1 |
| 59 | PRSS8 | protease, serine, 8 |
| 60 | RNASE7 | ribonuclease, RNase A family, 7 |
| 61 | SDC1 | syndecan 1 |
| 62 | SMPD1 | sphingomyelin phosphodiesterase 1, acid lysosomal |
| 63 | STS | steroid sulfatase (microsomal), isozyme S |
| 64 | TGM1 | transglutaminase 1 (K polypeptide epidermal type I, protein-glutamine-gamma-glutamyltransferase) |
| 65 | TGM5 | transglutaminase 5 |
| 66 | TJP1 | tight junction protein 1 (zona occludens 1) |
| 67 | TJP2 | tight junction protein 2 (zona occludens 2) |
| 68 | VCL | vinculin |
| 69 | CTNNB1 | catenin (cadherin-associated protein), beta 1, 88kDa |
| 70 | CCND1 | cyclin D1 |
| 71 | CDKN1A | cyclin-dependent kinase inhibitor 1A (p21, Cip1) |
| 72 | CDKN2A | cyclin-dependent kinase inhibitor 2A (melanoma, p16, inhibits CDK4) |
| 73 | MDM2 | Mdm2 p53 binding protein homolog (mouse) |
| 74 | RB1 | retinoblastoma 1 |
| 75 | APAF1 | apoptotic peptidase activating factor 1 |
| 76 | BAD | BCL2-associated agonist of cell death |
| 77 | BAX | BCL2-associated X protein |
| 78 | BCL2 | B-cell CLL/lymphoma 2 |
| 79 | BID | BH3 interacting domain death agonist |
| 80 | BIRC7 | baculoviral IAP repeat-containing 7 |
| 81 | CASP3 | caspase 3, apoptosis-related cysteine peptidase |
| 82 | CASP8 | caspase 8, apoptosis-related cysteine peptidase |
| 83 | CASP9 | caspase 9, apoptosis-related cysteine peptidase |
| 84 | DEDD | death effector domain containing |
| 85 | MT2A | metallothionein 2A |
| 86 | TGM2 | transglutaminase 2 (C polypeptide, protein-glutam ine-gamma-glutamyltransferase) |
| 87 | ANXA1 | annexin A1 |
| 88 | BTC | betacellulin |
| 89 | MKI67 | antigen identified by monoclonal antibody Ki-67 |
| 90 | PLA2G2D | phospholipase A2, group IID |
| 91 | PLCB3 | phospholipase C, beta 3 (phosphatidyl inositol-specific) |
| 92 | PLCG1 | phospholipase C, gamma 1 |
| 93 | PMAIP1 | phorbol-12-myristate-13-acetate-induced protein 1 |
| 94 | PTAFR | platelet-activating factor receptor |
| 95 | TGFA | transforming growth factor, alpha |
| 96 | ANXA5 | annexin A5 |
| 97 | ID1 | inhibitor of DNA binding 1, dominant negative helix-loop-helix protein |
| 98 | PLD2 | phospholipase D2 |
| 99 | PDGFRA | platelet-derived growth factor receptor, alpha polypeptide |
| 100 | PDGFRB | platelet-derived growth factor receptor, beta polypeptide |
| 101 | HAS1 | hyaluronan synthase 1 |
| 102 | HAS2 | hyaluronan synthase 2 |
| 103 | HAS3 | hyaluronan synthase 3 |
| 104 | SPTLC2 | serine palmitoyltransferase, long chain base subunit 2 |
| 105 | SPTLC3 | serine palmitoyltransferase, long chain base subunit 3 |
| 106 | CCL2 | chemokine (C-C motif) ligand 2 |
| 107 | CCL5 | chemokine (C-C motif) ligand 5 |
| 108 | CXCL1 | chemokine (C-X-C motif) ligand 1 (melanoma growth stimulating activity, alpha) |
| 109 | CXCL10 | chemokine (C-X-C motif) ligand 10 |
| 110 | PPARGC1A | peroxisome proliferator-activated receptor gamma, coactivator 1 alpha |
| 111 | TBXAS1 | thromboxane A synthase 1 (platelet) |
| 112 | TERF1 | telomeric repeat binding factor (NIMA-interacting) 1 |
| 113 | TERF2 | telomeric repeat binding factor 2 |
| 114 | EVPL | envoplakin |
| 115 | IVL | involucrin |
| 116 | KLF4 | Kruppel-like factor 4 (gut) |
| 117 | LCE2B | late cornified envelope 2B |
| 118 | LOR | loricrin |
| 119 | PPL | periplakin |
| 120 | RPTN | Repetin |
| 121 | TCHH | Trichohyalin |
| 122 | TGM3 | transglutaminase 3 (E polypeptide, protein-glutamine-gamma-glutamyltransferase) |
| 123 | CLU | clusterin |
| 124 | GADD45G | growth arrest and DNA-damage-inducible, gamma |
| 125 | HMOX1 | heme oxygenase (decycling) 1 |
| 126 | IGFBP3 | insulin-like growth factor binding protein 3 |
| 127 | TXN | thioredoxin |
| 128 | TXNRD1 | thioredoxin reductase 1 |
| 129 | TXNRD2 | thioredoxin reductase 2 |
| 130 | PTGER3 | prostaglandin E receptor 3 (subtype EP3) |
| 131 | PTGFR | prostaglandin F receptor (FP) |
| 132 | COL7A1 | collagen, type VII, alpha 1 |
| 133 | CRNN | cornulin |
| 134 | CST3 | cystatin C |
| 135 | CST6 | cystatin E/M |
| 136 | CTSD | cathepsin D |
| 137 | CTSL2 | cathepsin L2 |
| 138 | DSC1 | desmocollin 1 |
| 139 | DSG1 | desmoglein 1 |
| 140 | DSG2 | desmoglein 2 |
| 141 | DSG3 | desmoglein 3 (pemphigus vulgaris antigen) |
| 142 | KLK5 | kallikrein-related peptidase 5 |
| 143 | KLK7 | kallikrein-related peptidase 7 |
| 144 | PI3 | peptidase inhibitor 3, skin-derived |
| 145 | SLPI | secretory leukocyte peptidase inhibitor |
| 146 | SPINK5 | serine peptidase inhibitor, Kazal type 5 |
| 147 | CALML5 | calmodulin-like 5 |
| 148 | CRABP2 | cellular retinoic acid binding protein 2 |
| 149 | GDF15 | growth differentiation factor 15 |
| 150 | HBEGF | heparin-bindinq EGF-like growth factor |
| 151 | HOPX | HOP homeobox |
| 152 | ID2 | inhibitor of DNA binding 2, dominant negative helix-loop-helix protein |
| 153 | NR1H3 | nuclear receptor subfamily 1, group H, member 3 |
| 154 | NR1/2 | nuclear receptor subfamily 1, group I, member 2 |
| 155 | PPARA | peroxisome proliferator-activated receptor alpha |
| 156 | PPARG | peroxisome proliferator-activated receptor gamma |
| 157 | SPRR1A | small proline-rich protein 1A |
| 158 | SPRR1B | small proline-rich protein 1B (cornifin) |
| 159 | SPRR2A | small proline-rich protein 2A |
| 160 | SUMO2 | SMT3 suppressor of mif two 3 homolog 2 (S. cerevisiae) |
| 161 | SUMO3 | SMT3 suppressor of mif two 3 homolog 3 (S. cerevisiae) |
| 162 | TP63 | tumor protein p63 |
| 163 | VDR | vitamin D (1,25-dihydroxyvitamin D3) receptor |
| 164 | ATM | ataxia telangiectasia mutated |
| 165 | ATR | ataxia telangiectasia and Rad3 related |
| 166 | GADD45A | growth arrest and DNA-damage-inducible, alpha |
| 167 | OGG1 | 8-oxoguanine DNA glycosylase |
| 168 | PCNA | proliferating cell nuclear antigen |
| 169 | XPC | xeroderma pigmentosum, complementation group C |
| 170 | ACO1 | aconitase 1, soluble |
| 171 | ACSL5 | acyl-CoA synthetase long-chain family member 5 |
| 172 | CYB5R1 | cytochrome b5 reductase 1 |
| 173 | ENO1 | enolase 1, (alpha) |
| 174 | G6PD | glucose-6-phosphate dehydrogenase |
| 175 | GABPA | GA binding protein transcription factor, alpha subunit 60kDa |
| 176 | HADHB | hydroxyacyl-Coenzyme A dehydrogenase/3-ketoacyl-Coenzyme A thiolase/enoyl-Coenzyme A hydratase (trifunctional protein), beta subunit |
| 177 | KL | klotho |
| 178 | NDUFV2 | NADH dehydrogenase (ubiquinone) flavoprotein 2, 24kDa |
| 179 | OGDH | oxoglutarate (alpha-ketoglutarate) dehydrogenase (lipoamide) |
| 180 | PKM2 | pyruvate kinase, muscle |
| 181 | SOD1 | superoxide dismutase 1, soluble |
| 182 | SOD2 | superoxide dismutase 2, mitochondrial |
| 183 | P2RY2 | purinergic receptor P2Y, G-protein coupled, 2 |
| 184 | PLA2G5 | phospholipase A2, group V |
| 185 | PLA2G7 | phospholipase A2, group VII (platelet-activating factor acetylhydrolase, plasma) |
| 186 | CAV1 | caveolin 1, caveolae protein, 22kDa |
| 187 | COL4A4 | collaqen, type IV, alpha 4 |
| 188 | FMOD | fibromodulin |
| 189 | MATN2 | matrilin 2 |
| 190 | SPARC | secreted protein, acidic, cysteine-rich (osteonectin) |
| 191 | TNC | tenascin C |
| 192 | c-myc/MYC | v-myc myelocytomatosis viral oncogene |
| | | homolog (avian) |
| 193 | DLX3 | distal-less homeobox 3 |
| 194 | EGR1 | early growth response 1 |
| 195 | FOS | FBJ murine osteosarcoma viral oncogene homolog |
| 196 | JUN | jun oncogene |
| 197 | JUND | jun D proto-oncogene |
| 198 | NFKB1 | nuclear factor of kappa light polypeptide gene enhancer in B-cells 1 |
| 199 | NFKB2 | nuclear factor of kappa light polypeptide gene enhancer in B-cells 2 (p49/p100) |
| 200 | POU2F1 | POU class 2 homeobox 1 |
| 201 | EGF | epidermal growth factor (beta-urogastrone) |
| 202 | IGF1 | insulin-like growth factor 1 (somatomedin C) |
| 203 | VEGF/FLT1 | fms-related tyrosine kinase 1 (vascular endothelial growth factor/vascular permeability factor receptor) |
| 204 | SIRT1 | sirtuin (silent mating type information regulation 2 homolog) 1 (S. cerevisiae) |
| 205 | AANAT | arylalkylamine N-acetyltransferase |
| 206 | ASMT | acetylserotonin O-methyltransferase |
| 207 | CREB1 | cAMP responsive element binding protein 1 |
| 208 | CREBBP | CREB binding protein |
| 209 | CYP19A1 | cytochrome P450, family 19, subfamily A, polypeptide 1 |
| 210 | HSD17B2 | hydroxysteroid (17-beta) dehydrogenase 2 |
| 211 | HTR2A | 5-hydroxytryptamine (serotonin) receptor 2A |
| 212 | HTR2B | 5-hydroxytryptamine (serotonin) receptor |
| | | 2B |
| 213 | MTNR1A | melatonin receptor 1A |
| 214 | MTNR1B | melatonin receptor 1B |
| 215 | NR3A2/ESR2 | estrogen receptor 2 (ER beta |
| 216 | NR3C4/AR | androgen receptor |
| 217 | PRLR | prolactin receptor |
| 218 | RORA | RAR-related orphan receptor A |
| 219 | RORB | RAR-related orphan receptor B |
| 220 | SRD5A1 | steroid-5-alpha-reductase, alpha polypeptide 1 (3-oxo-5 alpha-steroid delta 4-dehydrogenase alpha 1) |
| 221 | SRD5A2 | steroid-5-alpha-reductase, alpha polypeptide 2 (3-oxo-5 alpha-steroid delta 4-dehydrogenase alpha 2) |
| 222 | TPH1 | tryptophan hydroxylase 1 |
| 223 | TPH2 | tryptophan hydroxylase 2 |
| 224 | B2M | beta-2-microglobulin |
| 225 | POLR2A | polymerase (RNA) II (DNA directed) polypeptide A, 220kDa |
| 226 | PPIA | peptidylprolyl isomerase A (cyclophilin A) |
| 227 | RPLP0, RPLP0P6 | ribosomal protein, large, P0, ribosomal protein, large, P0 pseudogene 6 |
| 228 | AQP3, | aquaporin 3 (Gill blood group) |
| 229 | AQP8, | aquaporin 8 |
| 230 | CD44 | CD44 molecule (Indian blood group) |
| 231 | CTSB | cathepsin B |
| 232 | CTSE | cathepsin E |
| 233 | CTSL1 | cathepsin L1 |
| 234 | HAL | histidine ammonia-lyase |
| 235 | PADI1 | peptidyl arginine deiminase, type I |
| 236 | PADI2 | peptidyl arginine deiminase, type II |
| 237 | PADI3 | peptidyl arginine deiminase, type III |
| 238 | CAPN1 | calpain 1, (mu/l) large subunit |
| 239 | CASP14 | caspase 14, apoptosis-related cysteine peptidase |
| 240 | ELF3 | E74-like factor 3 (ets domain transcription factor, epithelial-specific) |
| 241 | FLG | filaggrin |
| 242 | FURIN | furin (paired basic amino acid cleaving enzyme) |
| 243 | PPP2R2C | protein phosphatase 2 (formerly 2A), regulatory subunit B, gamma isoform |
| 244 | ST14 | suppression of tumorigenicity 14 (colon carcinoma) |
| 245 | IL11 | interleukin 11 |
| 246 | IL13 | interleukin 13 |
| 247 | IL18 | interleukin 18 (interferon-gamma-inducing factor) |
| 248 | IL19 | interleukin 19 |
| 249 | TLR1 | toll-like receptor 1 |
| 250 | TLR10 | toll-like receptor 10 |
| 251 | TLR2 | toll-like receptor 2 |
| 252 | TLR4 | toll-like receptor 4 |
| 253 | TLR5 | toll-like receptor 5 |
| 254 | TLR6 | toll-like receptor 6 |
| 255 | TLR9 | toll-like receptor 9 |
| 256 | TNFRSF10A | tumor necrosis factor receptor superfamily, member 10a |
| 257 | TNFRSF10B | tumor necrosis factor receptor superfamily, member 10b |
| 258 | BDKRB1 | bradykinin receptor B1 |
| 259 | CASP1 | caspase 1, apoptosis-related cysteine peptidase (interleukin 1, beta, convertase) |
| 260 | CCL27 | chemokine (C-C motif) ligand 27 |
| 261 | CCR10 | chemokine (C-C motif) receptor 10 |
| 262 | CHUK | conserved helix-loop-helix ubiquitous kinase |
| 263 | ICAM1 | intercellular adhesion molecule 1 |
| 264 | IFNG | interferon, gamma |
| 265 | IL10 | interleukin 10 |
| 266 | IL17A | interleukin 17A |
| 267 | IL1A | interleukin 1, alpha |
| 268 | IL1B | interleukin 1, beta |
| 269 | IL1R1 | interleukin 1 receptor, type I |
| 270 | IL1R2 | interleukin 1 receptor, type II |
| 271 | IL21R | interleukin 21 receptor |
| 272 | IL22RA1 | interleukin 22 receptor, alpha 1 |
| 273 | IL6 | interleukin 6 (interferon, beta 2) |
| 274 | ITGA2 | integrin, alpha 2 (CD49B, alpha 2 subunit of VLA-2 receptor) |
| 275 | LTA | lymphotoxin alpha (TNF superfamily, member 1) |
| 276 | PDE4A | phosphodiesterase 4A, cAMP-specific (phosphodiesterase E2 dunce homolog, Drosophila) |
| 277 | PLCE1 | phospholipase C, epsilon 1 |
| 278 | PTGDR | prostaglandin D2 receptor (DP) |
| 279 | PTGER2 | prostaglandin E receptor 2 (subtype EP2), 53kDa |
| 280 | PTGIS | prostaglandin I2 (prostacyclin) synthase |
| 281 | PYCARD | PYD and CARD domain containing |
| 282 | SOCS1 | suppressor of cytokine signaling 1 |
| 283 | STAT1 | signal transducer and activator of transcription 1, 91kDa |
| 284 | STAT3 | signal transducer and activator of transcription 3 (acute-phase response |
| | | factor) |
| 285 | TBXA2R | thromboxane A2 receptor |
| 286 | TLR3 | toll-like receptor 3 |
| 287 | TNF | tumor necrosis factor (TNF superfamily, member 2) |
| 288 | TNFRSF1A | tumor necrosis factor receptor superfamily, member 1A |
| 289 | TNFRSF1B | tumor necrosis factor receptor superfamily, member 1B |
| 290 | BDKRB2 | bradykinin receptor B2 |
| 291 | CB1/CNR1 | cannabinoid receptor 1 (brain) |
| 292 | CB2/CNR2 | cannabinoid receptor 2 (macrophage) |
| 293 | CGRPR/CALCRL | calcitonin receptor-like |
| 294 | CHRNA4 | cholinergic receptor, nicotinic, alpha 4 |
| 295 | IL8 | interleukin 8 |
| 296 | LTB4R | leukotriene B4 receptor |
| 297 | NK2/TACR2 | tachykinin receptor 2 |
| 298 | NK3/TACR3 | tachykinin receptor 3 |
| 299 | P2RX3 | purinergic receptor P2X, ligand-gated ion channel, 3 |
| 300 | TAC1 | tachykinin, precursor 1 |
| 301 | TACR1 | tachykinin receptor 1 |
| 302 | TRPV1 | transient receptor potential cation channel, subfamily V, member 1 |
| 303 | TRPV3 | transient receptor potential cation channel, subfamily V, member 3 |
| 304 | TRPV4 | transient receptor potential cation channel, subfamily V, member 4 |
| 305 | VPAC1/VIPR1 | vasoactive intestinal peptide receptor 1 |
| 306 | VPAC2/VIPR2 | vasoactive intestinal peptide receptor 2 |
| 307 | ACACA | acetyl-Coenzyme A carboxylase alpha |
| 308 | ASAH1 | N-acylsphingosine amidohydrolase (acid |
| | | ceramidase) 1 |
| 309 | HMGCR | 3-hydroxy-3-methylglutaryl-Coenzyme A reductase |
| 310 | PSAP | prosaposin |
| 311 | SGMS1 | sphingomyelin synthase 1 |
| 312 | SPTLC1 | serine palmitoyltransferase, long chain base subunit 1 |
| 313 | SREBF2 | sterol regulatory element binding transcription factor 2 |
| 314 | UGCG | UDP-glucose ceramide glucosyltransferase |
| 315 | GLB1 | galactosidase, beta 1 |
| 316 | ADAM9 | ADAM metallopeptidase domain 9 (meltrin gamma) |
| 317 | MMP1 | matrix metallopeptidase 1 (interstitial collagenase) |
| 318 | MMP10 | matrix metallopeptidase 10 (stromelysin 2) |
| 319 | MMP2 | matrix metallopeptidase 2 (gelatinase A, 72kDa gelatinase, 72kDa type IV collagenase) |
| 320 | MMP3 | matrix metallopeptidase 3 (stromelysin 1, progelatinase) |
| 321 | MMP9 | matrix metallopeptidase 9 (gelatinase B, 92kDa gelatinase, 92kDa type IV collagenase) |
| 322 | SERPINE1 | serpin peptidase inhibitor, clade E (nexin, plasminogen activator inhibitor type 1), member 1 |
| 323 | TIMP1 | TIMP metallopeptidase inhibitor 1 |
| 324 | TIMP2 | TIMP metallopeptidase inhibitor 2 |
| 325 | TMPRSS6 | transmembrane protease, serine 6 |
| 326 | PLCB1 | phospholipase C, beta 1 |
| | | (phosphoinositide-specific) |
| 327 | POMC | proopiomelanocortin |
| 328 | F2RL1 | coagulation factor II (thrombin) receptor-like 1 |
| 329 | FGFR2 | fibroblast growth factor receptor 2 |
| 330 | GPNMB | glycoprotein (transmembrane nmb |
| 331 | DRD2 | dopamine receptor D2 |
| 332 | MC1R | melanocortin 1 receptor (alpha melanocyte stimulating hormone receptor) |
| 333 | OPRM1 | opioid receptor, mu 1 |
| 334 | PCSK1 | proprotein convertase subtilisin/kexin type 1 |
| 335 | PCSK2 | proprotein convertase subtilisin/kexin type 2 |
| 336 | PCSK6 | proprotein convertase subtilisin/kexin type 6 |
| 337 | PTGS1 | prostaglandin-endoperoxide synthase 1 (prostaglandin G/H synthase and cyclooxygenase) |
| 338 | PTGS2 | prostaglandin-endoperoxide synthase 2 (prostaglandin G/H synthase and cyclooxygenase) |
| 339 | CANX | calnexin |
| 340 | HSP27/HSPB1 | heat shock 27kDa protein 1 |
| 341 | HSP27/HSPB2 | heat shock 27kDa protein 2 |
| 342 | HSP70/HSPA1B, HSPA1A | heat shock 70kDa protein 1B,heat shock 70kDa protein 1A |
| 343 | HSP90AA1 | heat shock protein 90kDa alpha (cytosolic), class A member 1 |
| 344 | HSPBP1 | HSPA (heat shock 70kDa) binding protein, cytoplasmic cochaperone 1 |
| 345 | ATF2 | activating transcription factor 2 |
| 346 | EGFR | epidermal growth factor receptor (erythroblastic leukemia viral (v-erb-b) oncogene homolog, avian) |
| 347 | HPSE | heparanase |
| 348 | MAX | MYC associated factor X |
| 349 | NFKBIZ | nuclear factor of kappa light polypeptide gene enhancer in B-cells inhibitor, zeta |
| 350 | PLCB2 | phospholipase C, beta 2 |
| 351 | TGFB1 | transforming growth factor, beta 1 |
| 352 | TGFBR1 | transforming growth factor, beta receptor 1 |
| 353 | TP53 | tumor protein p53 |
| 354 | XPA | xeroderma pigmentosum, complementation group A |
| 355 | POT1 | POT1 protection of telomeres 1 homolog (S. pombe) |
| 356 | TERT | telomerase reverse transcriptase |
| 357 | CSF2 | colony stimulating factor 2 (granulocyte-macrophage) |
| 358 | EDN1 | endothelin 1 |
| 359 | EDNRB | endothelin receptor type B |
| 360 | FGFR1 | fibroblast growth factor receptor 1 |
| 361 | NGF | nerve growth factor (beta polypeptide) |
| 362 | PDGFA | platelet-derived growth factor alpha polypeptide |
| 363 | PDGFB | platelet-derived growth factor beta polypeptide (simian sarcoma viral (v-sis) oncogene homolog) |
| 364 | PLA2G10 | phospholipase A2, group X |
| 365 | PLA2G2A | phospholipase A2, group IIA (platelets, synovial fluid) |
| 366 | PPARD | peroxisome proliferator-activated receptor delta |
| 367 | PTGES2 | prostaglandin E synthase 2 |
| 368 | RXFP1 | relaxin/insulin-like family peptide receptor 1 |
| 369 | RXFP2 | relaxin/insulin-like family peptide receptor 2 |
| 370 | SORBS3 | sorbin and SH3 domain containing 3 |
| 371 | VEGFA | vascular endothelial growth factor A |
| 372 | VEGFB | vascular endothelial growth factor B |
| 373 | VEGFC | vascular endothelial growth factor C |
| 374 | VEGFD/FIGF | c-fos induced growth factor (vascular endothelial growth factor D) |

The compositions of the present invention have attributes of interest for the treatment of keratinous materials as can be seen below in the examples presented.

The following examples illustrate the compositions, methods and use of the present invention without representing any limitation thereto. The rates indicated are percentages by weight.

### Examples

The expression of genes related to the ROS metabolism, lipid metabolism, skin integrity, cell renewal, cell differentiation, strengthening of the skin barrier, calcium metabolism, cell proliferation, tissue healing and/or repair, cell adhesion, carbohydrate metabolism, hydration, skin homeostasis/maintenance, skin defense and/or protection, remodeling and/or extracellular matrix synthesis, skin shedding, tissue maintenance, cell signalling, normal development of the epidermis, epidermal barrier function, skin development, the dermis-epidermis junction, anti-inflammatory or antioxidant action was tested in a reconstructed human epidermis culture. The analysis was performed in vitro using 3D culture models of reconstructed human epidermis. The cultures (0.63 cm2) were maintained in appropriate media for 14 days in greenhouse at 37°C with 5% CO2.

### Analysis of gene expression by qRT-PCR TaqMan arrays:

The analysis of gene expression by qRT-PCR compares the expression level of one or more genes between samples under different treatments with plant lipids in order to evaluate the influence of these treatments on the expression of these genes.

The analysis of the expression of 384 genes (Table 1) was performed in cultures of reconstructed human epidermis subjected to treatment with different raw materials (n=3), which were diluted in mineral oil, which was used as carrier.

The ΔCt values for the reference gene (18S ribosomal RNA) and target gene were calculated by subtracting the values from the experimental groups (treated with mineral oil or treated with the plant lipid diluted in mineral oil).

Subsequently, the ΔCt of the experimental group (culture treated with the plant lipid) was subtracted from the control group (culture treated mineral oil) for obtaining ΔΔCt. Finally, the relative quantification of the target genes was determined by the equation: RQ = 2-ΔΔCt. Only values > 2.0 and < 0.5 were considered in subsequent analyzes. The statistical significance was assessed by t-test, with p-value < 0.05 being considered significant.

The following plant lipids were assessed: acai oil, andiroba oil, buriti oil, passion fruit oil, palm olein, cupuacu butter, cocoa butter, murumuru butter, pataua oil, ucuuba butter.

### Analysis in the Ingenuity Pathway Analysis Software (IPA)

The values detected as statistically significant were loaded into the Ingenuity Pathway Analysis software (IPA) (http://www.ingenuity.com), to investigate the functional relationships between the identified genes. For each pathway/network established, a p-score [p-score = - log10 (p-value)] was generated reflecting the probability of this network be generated at random and where the p-value was calculated by Fisher's exact test. This means that if a pathway has a p-score of 10, the chance that this network is generated at random is less than 1 in 10¹⁰.

These results show the genes and functions modulated by the compositions and methods of the present invention, where the following Examples 1 (where for use in tissue healing and/or repair), 11 and 12 are examples of the invention, and Examples 2 to 10 are reference examples.

### Example 1: Acai oil

The cosmetic and pharmaceutical compositions containing acai oil were tested with respect to the functioning and/or maintenance of keratinous materials, especially their performance with the modulation of gene *expression. A positive result was verified for the following functions:*

| **Modulated functions** | **Induced Genes** | **Repressed Genes** |
|---|---|---|
| ROS Metabolism | ANXA1, BID, CASP3, CAV1, EDN1, EGFR, F2RL1, GPX1, HMOX1, ITGA6, ITGB1, ITGB4, PTGS2, RB1, SIRT1, SOD2, TGFA, TGFB1, TGM2, TLR2, TNF, TXN, MMP3, MYC, VEGFA | |
| Lipid Metabolism | CLU, CXCL1, DEFB4A/DEFB4B, EDN1, EGFR, IL1R2, MYC, NFKB1, NR1H2, NR1H3, PLA2G10, PTGS2, SIRT1, TGFA, TGFB1, TLR2, TNF, VEGFA, CAV1, ITGA6, ITGB1, PDGFA, PPARA, SPTLC1, STS, UGCG, PPARD, ANXA1, SRD5A1, BID, GABPA, HSD17B2, TBXAS, BDKRB2, VDR, MMP3 | |
| Skin Integrity | CDSN, COL17A1, COL7A1, CTSL1, FLG, ITGA2, KLK5, KRT14, KRT15, KRT17, KRT5, LAMB3, PPARD, STS, UGCG, VEGFA, ANXA1, CTNNB1, EDN1, ID1, ITGB1, IVL, LOR, MYC, TGFA, TGFB1, TNF, TP63, CXCL10, TGFBR1, EGFR, HBEGF | |
| Cell Renewal | BTC, EGFR, GDF15, HBEGF, IL11, LAMA5, MYC, RB1, TGFA, TGFB1, TGM2, TP63, CLU, EDN1, ID1, ITGB1, PPARD, SPARC, TLR2, TNF, | |
| | VEGFA, CDKN2A, CTNNB1, MMP2, PDGFA, SOD2, TIMP1, TNFRSF10B | |
| Cell Differentiation | ANXA1, CREBBP, CTNNB1, EDN1, EGFR, FLG, ID1, ITGB1, IVL, KLF4, LOR, MYC, PPARD, TGFA, TGFB1, TIMP1, TIMP2, TNF, TP63, HBEGF | |
| Strengthening the Skin Barrier | LCE2B, CDSN, FURIN, ST14, BTC, LOR, PADI1, CST6, KLK5, ITGB6 | |
| Calcium Metabolism | ANXA1, CAV1, CXCL1, CXCL10, DEFB103A/DEFB103B, DEFB4A/DEFB4B, EDN1, F2RL1, MMP1, P2RY2, TGFB1, TLR2, TNF, TRPV4, VEGFA, CCL27, CD44, EGFR, LTB4R, EDN1, TGFA, VIPR1 | CALCRL |
| Cell Proliferation | BTC, EGFR, GDF15, HBEGF, IL11, LAMA5, MYC, RB1, TGFA, TGFB1, TGM2, TP63, CLU, EDN1, ID1, ITGB1, PPARD, SPARC, TLR2, TNF, VEGFA, CDKN2A, CTNNB1, MMP2, PDGFA, SOD2, TIMP1, TNFRSF10B | |
| Tissue Healing or Repair | PPARD, HMOX1, BTC, MMP1, MMP2, MMP3 | |
| Cell Adhesion | COL7A1, ITGA6, ITGB1, TNC, TNF, VCL, CLU, EGFR, HBEGF, PI3, SOD2, TIMP1, F2RL1, TGFA, CAV1, MYC, VEGFA | |
| Carbohydrate Metabolism | CD44, CXCL10, TGFB1, TLR2, TNC, TNF, VEGFA, KRT14, CTNNB1, EDN1, HAS3, HBEGF, HPSE, IGF1R, IGFBP3, IL1R2, ITGB1, P2RY2, PDGFA, PLA2G10, PPARA, PTGS2 | |
| Hydration | HAS3, FURIN, PADI1 | |
| Skin Homeostasis | BTC, TIMP2, MT2A, CTSE, ITGA6, CTSL2, TRPV4 | |
| Skin Defense and Protection | DEFB103A, DEFB103B, RNASE7 | |
| Extracellular Matrix Synthesis | HAS3, COL17A1, COL7A1, SPARC | |
| Skin Peeling | CTSL2, CST6, KLK5 | |

### Example 2: Buriti oil

The cosmetic and pharmaceutical compositions containing buriti oil were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Cell Renewal | CLU, EDN1, HBEGF, IL8, SPARC, TGFA, TNF, BTC, GDF15, ID2 | |
| Cell Differentiation | EDN1, FLG, KLF4, LOR, TGFA, TIMP2, TNF | |
| Cell Proliferation | CLU, EDN1, HBEGF, IL8, SPARC, TGFA, TNF, BTC, GDF15, ID2 | |
| Lipid Metabolism | CLU, CXCL1, EDN1, IL1R2, IL8, PTGS2, SIRT1, TGFA, TNF, UGCG, VIPR1, HMOX1, ITGA6 | |
| Strengthening the Skin Barrier | KLK5, CDSN, LCE2B, FLG, LOR, KRT4 | |
| ROS Metabolism | CCL2, IL19, IL8, ITGA6, TNF, JUN, MMP3, EDN1, HMOX1, PTGS2, SIRT1, TGFA | |
| Skin Integrity | CDSN, COL7A1, FLG, IL19, KLK5, UGCG, EDN1, LOR, TGFA, TNF, CLU, IL8 | |
| Calcium Metabolism | CCL2, EDN1, TGFA, TNF, VIPR1, | |
| | CXCL1, IL8 | |
| Cell Adhesion | HBEGF, IL8, TGFA, TNF, COL7A1, ITGA6, CLU, | SLPI |
| Skin Homeostasis | GDF15, TIMP2 | |
| Tissue Healing or Repair | EDN1, HMOX1, IL8, JUN, LTA, TGFBR1, TIMP2, TNF, CDSN, COL7A1, FLG, IL19, KLK5, UGCG, CCL2, ITGA6, CLU, HBEGF, FOS, TGFA | SLPI |
| Skin Peeling | CST6 | |
| Carbohydrate Metabolism | LTA, TNF, CXCL1, EDN1, IL8 | |

### Example 3: Passion fruit oil

The cosmetic and pharmaceutical compositions containing passion fruit oil were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Cell Differentiation | FLG, LOR, TIMP2 | PPARG |
| Tissue Maintenance | GDF15, TIMP2 | |
| Cell Signaling | | PPARG |
| Strengthening the Skin Barrier | FLG, LOR, LCE2B, PADI1, KRT4 | |
| Cell Adhesion | GDF15, SPARC, CDSN | |
| Skin Homeostasis | GDF15, TIMP2 | |
| Tissue Healing or Repair | FMOD, BTC | |

### Example 4: Palm olein

The cosmetic and pharmaceutical compositions containing palm olein were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Cell Differentiation | LCE2B | |
| Carbohydrate Metabolism | | MMP1, MMP10 |
| Calcium Metabolism | | CCR10 |
| Strengthening the Skin Barrier | LCE2B | |
| Cell Proliferation | BTC | |
| Reduction of Extracellular Matrix Degradation | | MMP10, MMP1 |
| Skin Defence/Protection | RNASE7 | |
| Tissue Repair | BTC, LCE2B | |
| Antiinflamatory | | IL1B |

### Example 5: Cocoa butter

The cosmetic and pharmaceutical compositions containing cocoa butter were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Normal Development of the Epidermis | CDSN, COL17A1, COL7A1, FLG, KLK5, KRT15, KRT5, LAMB3, STS, VEGFA, ANXA1, LOR, TGFB1, ITGB1, ITGB4 | CDH1, DSG3, DSP, JUP, KRT10 |
| Lipid Metabolism | CAV1, CXCL1, DEFB4A/DEFB4B, IL1R2, ITGA6, ITGB1, PDGFA, | G6PD, GLB1, PLA2G7, PLD2, PTGS1, SOD1 |
| | PLA2G10, PTAFR, PTGS2, STS, TGFB1, VEGFA, ANXA1, DRD2, VDR, VIPR1, APAF1, HMOX1, | |
| Carbohydrate Metabolism | HAS3, IGF1R, IGFBP3, IL1R2, ITGB1, PDGFA, PLA2G10, PTAFR, PTGS2, TGFB1, VEGFA, MMP2, VIPR1 | GLB1, PLA2G7, G6PD |
| Cell Differentiation | FLG, ITGB1, LOR, TGFB1, TIMP2 | CDH1, DSP |
| Skin Integrity | CDSN, COL17A1, COL7A1, FLG, KLK5, KRT15, KRT5, LAMB3, STS, VEGFA, ANXA1, ITGB1, LOR, TGFB1, ITGB4 | DSG3, CDH1, DSP, JUP, KRT10 |
| ROS Metabolism | ANXA1, CAV1, HMOX1, ITGA6, ITGB1, ITGB4, PTGS2, TGFB1, TGM2 | PTGS1, SOD1 |
| Calcium Metabolism | ANXA1, CAV1, CXCL1, DEFB4A/DEFB4B, DRD2, MMP1, TGFB1, TRPV4, VEGFA | DEFB1, SOD1 |
| Strengthening the Skin Barrier | ITGB1, CDSN, CRNN, TGFB1, ITGA6, ITGB4, COL7A1, CAV1, VEGFA, LCE2B, KLK5, LOR, PADI1 | DSG3, CDH1, JUP |
| Cell Renewal | ITGB1, PTAFR, SPARC, TGFB1, VEGFA, BTC, IL11, TGM2 | STAT3 |
| Tissue Healing and/or Repair | HMOX1, BTC, MMP3, FMOD, AR | |
| Cell Proliferation | ITGB1, PTAFR, SPARC, TGFB1, VEGFA, BTC, IL11, TGM2 | STAT3 |
| Hydration | HAS3, PADI1, TGM2 | |
| Remodeling/Extracellular Matrix Synthesis | MMP3, TIMP2, HAS3, KLK5, SERPINE1 | |

### Example 6: Andiroba oil

The cosmetic and pharmaceutical compositions containing andiroba oil were tested with respect to the functioning and/or maintenance of keratinous materials, especially their performance with the modulation of gene expression. A positive result was verified for the following functions:

| **Modulated Functions** | **Induced Genes** |
|---|---|
| Lipid Metabolism | CAV1, CLU, CXCL1, DEFB4A/DEFB4B, EGFR, GBA, IL1R2, ITGA6, ITGB1, NFKB1, NR1H3, PDGFA, PPARA, PTAFR, PTGS2, SIRT1, STS, TGFA, TGFB1, UGCG, VEGFA, PPARD, ANXA1, SRD5A1, APAF1, DRD2, HMOX1, BDKRB2, VDR |
| Skin Integrity | CDSN, COL17A1, COL7A1, CTSL1, FLG, ITGA2, KLK5, KRT14, KRT15, KRT17, KRT5, LAMB3, PPARD, STS, UGCG, VEGFA, ANXA1, CTNNB1, ITGB1, IVL, LOR, TGFA, TGFB1, TP63, CLU, EGFR, PTAFR |
| Cell Renewal | BTC, EGFR, IL11, RB1, TGFA, TGFB1, TGM2, TP63, CLU, ITGB1, PPARD, PTAFR, SPARC, VEGFA, CTNNB1, MMP2, PDGFA, SOD2, TIMP1, TNFRSF10B |
| Carbohydrate Metabolism | CTNNB1, HAS3, IGF1R, IGFBP3, IL1R2, ITGB1, P2RY2, PDGFA, PTAFR, PTGS2, |
| | TGFB1, VEGFA, HPSE, CASP3, GBA, ITGA2, |
| Cell Differentiation | ANXA1, CTNNB1, EGFR, FLG, ITGB1, IVL, KLF4, LOR, PPARD, TGFA, TGFB1, TIMP1, TIMP2, TP63 |
| ROS Metabolism | ANXA1, CASP3, CAV1, EGFR, F2RL1, GPX1, HMOX1, ITGA6, ITGB1, ITGB4, PTGS2, RB1, SIRT1, SOD2, TGFA, TGFB1, TGM2 |
| Cell Adhesion | COL7A1, ITGA6, ITGB1, TNC, VCL, CLU, EGFR, PI3, SOD2, TIMP1, CAV1, VEGFA, CDSN, CRNN, CTNNB1, TGFB1 |
| Strengthening the Skin Barrier | DRD2, KLK5, CST6, FURIN, LCE2B, LOR, PADI1, CDSN, IL1R2 |
| Cell Proliferation | BTC, EGFR, IL11, RB1, TGFA, TGFB1, TGM2, TP63, CLU, ITGB1, PPARD, PTAFR, SPARC, VEGFA, CTNNB1, MMP2, PDGFA, SOD2, TIMP1, TNFRSF10B |
| Hydration | PADI1, TGM2, HAS3 |
| Tissue Healing and/or Repair | HMOX1, MMP3, BTC, FMOD, AR |
| Skin Defense and/or Protection | KLK5, RNASE7, GPX1 |
| Peeling | KLK5, CST6 |
| Extracellular Matrix Synthesis | KLK5, TIMP2, HAS3 |

### Example 7: Cupuacu butter

The cosmetic and pharmaceutical compositions containing cupuacu butter were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Lipid Metabolism | CAV1, CXCL1, DEFB4A/DEFB4B, IL1R2, ITGA6, ITGB1, PDGFA, PTAFR, PTGS2, STS, TGFB1, VEGFA, ANXA1, HMOX1, APAF1, VIPR1 | CASP1, CAT, G6PD, GLB1, PLA2G7, PRDX6, PTGS1, SOD1, SPTLC3 |
| Development of the Skin | CDSN, COL17A1, COL7A1, FLG, KLK5, KRT15, KRT5, LAMB3, STS, VEGFA, ITGB1, LOR, TGFB1, ITGB4 | DSP, JUP, KRT10, DSG3 |
| ROS Metabolism | ANXA1, CAV1, F2RL1, HMOX1, ITGA6, ITGB1, ITGB4, PTGS2, TGFB1, TGM2, MMP3, VEGFA | SOD1, PTGS1, CAT, PRDX6 |
| Carbohydrate Metabolism | HAS3, IGFBP3, IL1R2, ITGB1, PDGFA, PTAFR, PTGS2, TGFB1, VEGFA, MMP2, | G6PD, PLA2G7 |
| Calcium Metabolism | ANXA1, CAV1, CXCL1, DEFB4A/DEFB4B, F2RL1, MMP1, TGFB1, TRPV4, VEGFA | DEFB1, SOD1 |
| Cell Differentiation | IGFBP3, LCE2B, CDSN, DSG2, BTC, LOR, PADI1, AR, MT2A | |
| Cell Proliferation | ITGB1, PTAFR, SPARC, TGFB1, VEGFA, BTC, IL11, TGM2 | STAT3 |
| Cell Adhesion | CAV1, ITGB1, VEGFA, ITGA6, ITGB4, CDSN, CRNN, TGFB1, COL7A1 | JUP, DSG3, CAT |
| Skin Homeostasis/Maintenance | MT2A, BTC, TIMP2, F2RL1, ITGA6, CTSL2 | |
| Strengthening the Skin Barrier | IGFBP3, LCE2B, CDSN, DSG2, BTC, LOR, PADI1, F2RL1 | |
| Healing | MT2A, MMP3, BTC, HMOX1, FMOD, AR | |
| Skin Hydration | HAS3, PADI1, TGM2 | |
| Dermis-Epidermis Junction | COL17A1, COL7A1, ITGA6, ITGB1, ITGB4, ITGB6 | |
| Extracellular Matrix Synthesis | HAS3, MMP3, TIMP2 | |

### Example 8: Murumuru butter

The cosmetic and pharmaceutical compositions containing murumuru butter were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Lipid Metabolism | CXCL1, DEFB4A/DEFB4B, IL1R2, PTAFR, PTGS2, SIRT1, TGFA, TGFB1, VEGFA, CAV1, ITGA6, ITGB1, PDGFA, ANXA1, PPARD, STS, HMOX, APAF1, CASP9, TBXAS1, BDKRB2, MMP3 | PLD2, PPARG, PTGS1 |
| Skin Maintenance | AR, CXCL1, F2RL1, ITGA6, ITGB4, PDGFA, TGFB1, VEGFA, VEGFC, DSC2, DSG2 | CDH1, PLD2, PPARG |
| Development of the Skin | CDSN, COL17A1, COL7A1, FLG, KLK5, KRT14, KRT15, KRT5, LAMB3, PPARD, STS, VEGFA, ANXA1, ITGB1, LOR, TGFA, TGFB1, ITGB4, PTAFR | DSP, KRT1, KRT10, CDH1 |
| ROS Metabolism | ANXA1, CAV1, F2RL1, HMOX1, ITGA6, ITGB1, ITGB4, PTGS2, SIRT1, TGFA, TGFB1,TGM2 | PPARG, PTGS1 |
| Calcium Metabolism | ANXA1, CAV1, CXCL1, DEFB103A/DEFB103B, DEFB4A/DEFB4B, F2RL1, MMP1, TGFB1, TRPV4, VEGFA, PTAFR, | CALCRL |
| Carbohydrate Metabolism | HAS3, IGFBP3, ITGB1, IL1R2, KRT14, TGFB1, VEGFA, CASP9 | |
| Cell Differentiation | ANXA1, FLG, ITGB1, LOR, PPARD, TGFA, TGFB1, TIMP2 | CDH1, DSP |
| Cell Proliferation | IGFBP3, PPARD, BTC, SERPINE1 | |
| Strengthening the Skin Barrier | LCE2B, LOR, PADI1, VEGFA | |
| Healing | HMOX1, PPARD, MMP3, BTC | |
| Cell Adhesion | ITGA6, ITGB1, ITGB4, CDSN, CRNN, TGFB1, COL7A1, CAV1, VEGFA | CDH1 |
| Skin Hydration | HAS3, PADI1, | |
| Skin Defense and Protection | APAF1, DEFB103A, DEFB103B, RNASE7 | |
| Extracellular Matrix Synthesis | HAS3, TIMP2 | |
| Skin Homeostasis/Maintenance | BTC, MT2A, TIMP2, GDF15, ITGA6 | |

### Example 9: Ucuuba butter

The cosmetic and pharmaceutical compositions containing ucuuba butter were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Lipid Metabolism | CAV1, CXCL1, DEFB4A/DEFB4B, IL1A, IL1R2, IL8, ITGA6, ITGB1, MYC, NFKB1, NGF, PLCB1, PTAFR, PTGS2, SIRT1, TGFA, TGFB, TNF, UGCG, VEGFA, PPARD, ANXA1, ANXA5, APAF1, CASP9, HMOX1, VIPR1, EGR1, VDR, TIMP1, APAF1, | GLB1, IL18, PDGFB, PTGS1, SREBF2 |
| Carbohydrate Metabolism | HAS1, HAS3, HBEGF, HPSE, IGFBP3, IL1A, IL1R2, IL8, ITGB1, NGF, PLCB1, PTAFR, PTGS2, TGFB1, TNF, VEGFA, KRT14, TNC, CXCL10, ANXA5, APAF1, CASP9, CAV1, MMP9, SOD2 | IL18, PDGFB |
| ROS Metabolism | ANXA1, CASP3, CAV1, F2RL1, HMOX1, IL8, ITGA6, ITGB1, ITGB4, PTGS2, SIRT1, SOD2, TGFA, TGFB1, TGM2, TNF, JUN, MMP3, MYC, NGF, VEGFA | IL18, PTGS1 |
| Development of the Skin | CDSN, COL17A1, COL7A1, CTSL1, FLG, ITGA2, KLK5, KRT14, KRT15, KRT16, KRT17, KRT5, LAMB3, PPARD, UGCG, VEGFA, ANXA1, IL1A, ITGB1, IVL, LOR, MYC, TGFA, TGFB1, TNF | KRT10 |
| Calcium Metabolism | ANXA1, CAV1, CXCL1, CXCL10, DEFB4A/DEFB4B, F2RL1, IL8, MMP1, TGFB1, TNF, TRPV4, VEGFA, LTB4R, NGF, PLCB1, PTAFR, ITGA2, ITGB1, | IL18, PDGFB |
| Cell Differentiation | ANXA1, FLG, IL1A, ITGB1, IVL, LOR, MYC, PPARD, TGFA, TGFB1, TNF, TIMP1, TIMP2 | |
| Cell Proliferation | HBEGF, IL8, ITGB1, MYC, PPARD, PTAFR, SPARC, TGFA, TGFB1, TNF, VEGFA, BTC, GDF15, IL11, TGM2 | |
| Strengthening the Skin Barrier | CST6, KLK5, IVL, LCE2B, LOR | |
| Healing | HMOX1, IL1A, PPARD | |
| Cell Adhesion | F2RL1, HBEGF, IL8, TGFA, TNF, COL7A1, ITGA6, ITGB1, TNC, CAV1, MYC, SOD2, VEGFA | |
| Skin Hydration | HAS3, HAS1, PADI1, TGM2 | |
| Skin Homeostasis/Maintenance | BTC, MT2A, GDF15, ITGA6, CTSL2 | |

### Example 10: Pataua oil

The cosmetic and pharmaceutical compositions containing pataua oil were tested with respect to the functioning and/or maintenance of keratinous materials, especially their performance with the modulation of gene expression. A positive result was verified for the following functions:

| **Modulated Functions** | **Induced Genes** | **Repressed Genes** |
|---|---|---|
| Carbohydrate Metabolism | HAS3, IL1R2 | |
| Strengthening the Skin Barrier | KLK5, CDSN, LCE2B, LOR | |
| Adhesion | CDSN | |
| Differentiation | CDSN, LCE2B, RNASE7 | |
| Development of the Skin | CDSN, BTC, ITGA6, LCE2B, LOR, TGFB1 | |
| Extracellular Matrix | HAS3 | MMP10 |
| Anti-inflammatory | | IL1A, IL1B, IL8 |
| Proliferation | SERPINE1 | |
| Skin Defense/Protection | RNASE7 | |
| Antioxidant Action | SERPINE1, HMOX1 | |

### Results

As a result, the attributes mentioned above were confirmed. The number of genes modulated by the different compositions tested in the project was also determined. This result is shown in Figure 1. Up arrows indicate the number of genes that had their expression increased in relation to the control group (induced genes), whereas down arrows indicate the number of genes that showed a reduction in their expression (repressed genes) in relation to the respective control.

### Example 11

### Shampoo composition

| **Function** | **Components** |
|---|---|
| **Surfactants** | Cocoamido Propyl Betaine Decyl Polyglucose Sodium Lauryl Ether Sulfate |
| **Chelating Agent** | EDTA |
| **Preservative** | Kathon |
| **Conditioner** | Dimethicone, Dimethiconol TEA |
| **Viscosity Agent** | Carbopol Aqua, Sodium Chloride |
| **Perfuming** | Fragrances |
| **Pearlizing** | Glycol distearate |
| **pH Correction** | Triethanolamine |

### Example 12

### Body Oil

| **Function** | **Components** |
|---|---|
| **Emollients** | Palm Olein Hexylene glycol |
| **Chelating Agent** | EDTA |
| **Preservative** | Kathon |
| **Viscosity Agent** | Sodium chloride |
| **Perfuming** | Fragrances |
| **Deodorant Active** | Sensiva |

## Claims

1. A composition for use in tissue healing and/or repair by modulating the expression of genes responsible for the functionality and maintenance of keratinous materials, wherein said composition comprises a raw material diluted in mineral oil, and a pharmaceutically and/or cosmetically acceptable carrier, wherein the raw material is a plant lipid and the plant lipid is acai oil.

2. The composition for use according to claim 1, **characterized in that** the plant lipid is present in an amount ranging from about 0.01% to about 50% by weight, preferably from about 0.5% to about 20% by weight, more preferably from about 1% to about 5% by weight, and the amounts are based on the total weight of the composition.

3. The composition for use according to any one of claims 1 to 2, **characterized in that** it is formulated for topical, oral or parenteral administration.

4. The composition for use according to claim 3, **characterized in that** it is in the form of aqueous, alcoholic or oily solutions, dispersions, emulsions, suspensions, aerosol compositions comprising a pressurized propellant agent, microcapsules, microparticles or vesicular dispersions of the ionic or non-ionic type, aqueous or oily lotions, serum, capsules, tablets, granules or syrups.

## Patentansprüche

1. Zusammensetzung zur Verwendung bei Gewebeheilung und/oder -reparatur durch Modulation der Expression von Genen, die für die Funktionalität und den Erhalt von keratinösen Materialien verantwortlich sind, wobei die Zusammensetzung ein Rohmaterial in Mineralöl gelöst und einen pharmazeutisch und/oder kosmetisch akzeptablen Trägerstoff umfasst, wobei das Rohmaterial ein Pflanzenlipid ist und das Pflanzenlipid Acai-Öl ist.

2. Zusammensetzung zur Verwendung nach Anspruch 1, **dadurch gekennzeichnet, dass** das Pflanzenlipid in einer Menge vorliegt, die von etwa 0,01 Gew.-% bis etwa 50 Gew.-% reicht, bevorzugt von etwa 0,5 Gew.-% bis etwa 20 Gew.-%, bevorzugter von etwa 1 Gew.-% bis etwa 5 Gew.-%, und die Mengen auf dem Gesamtgewicht der Zusammensetzung basieren.

3. Zusammensetzung zur Verwendung nach einem der Ansprüche 1 bis 2, **dadurch gekennzeichnet, dass** sie zur topischen, oralen oder parenteralen Verabreichung formuliert ist.

4. Zusammensetzung zur Verwendung nach Anspruch 3, **dadurch gekennzeichnet, dass** sie in der Form von wässrigen, alkoholischen oder öligen Lösungen, Dispersionen, Emulsionen, Suspensionen, Aerosol-Zusammensetzungen, die ein Treibmittel umfassen, Mikrokapseln, Mikropartikeln oder vesikulären Dispersionen vom ionischen oder nichtionischen Typ, wässrigen oder öligen Lotionen, Serum, Kapseln, Tabletten, Granulaten oder Sirups ist.

## Revendications

1. Composition destinée à être utilisée dans la cicatrisation et/ou la réparation tissulaire en modulant l'expression des gènes responsables de la fonctionnalité et du maintien de matières kératiniques, dans laquelle ladite composition comprend un matériau brute dilué dans de l'huile minérale, et un vecteur pharmaceutiquement et/ou cosmétiquement acceptable, dans laquelle le matériau brut est un lipide végétal et le lipide végétal est l'huile d'açaï.

2. Composition destinée à être utilisée selon la revendication 1, **caractérisée en ce que** le lipide végétal est présent en une quantité se situant dans la plage allant d'environ 0,01 % à environ 50 % en poids, de préférence d'environ 0,5 % à environ 20 % en poids, plus préférentiellement d'environ 1 % à environ 5 % en poids, et les quantités sont basées sur le poids total de la composition.

3. Composition destinée à être utilisée selon l'une quelconque des revendications 1 à 2, **caractérisée en ce qu'**elle est formulée pour une administration par voie topique, orale ou parentérale.

4. Composition destinée à être utilisée selon la revendication 3, **caractérisée en ce qu'**elle se présente sous la forme de solutions aqueuses, alcooliques ou huileuses, de dispersions, d'émulsions, de suspensions, de compositions d'aérosol comprenant un agent propulseur sous pression, de microgélules, de microparticules ou de dispersions vésiculaires de type ionique ou non ionique, de solutions aqueuses ou huileuses, de sérum, de gélules, de comprimés, de granules ou de sirops.
